# EUROPEAN PATENT APPLICATION

(11) **EP 4 643 843 A1**
(43) Date of publication of application: **05.11.2025**
(21) Application number: 25153298.2
(22) Date of filing: 22.01.2025
(51) Int. Cl.: A61K 8/06, A61K 8/34, A61K 8/37, A61K 8/49, A61K 8/63, A61K 8/73, A61K 8/97, A61Q 19/00, A61Q 19/08

(54) **A COSMETIC OIL IN WATER EMULSION COMPOSITION COMPRISING APPLE JUICE**

(30) Priority: 30.04.2024 PL 44845924
(71) Applicant: Ledniowski, Dariusz, 33-386 Podegrodzie (PL)
(72) Inventor: Bochenek, Monika, 33-386 Cracow (PL); Budzisz, Elzbieta, 90-419 Lodz (PL); Mucha, Paulina, 90-419 Lodz (PL)
(74) Representative: Kondrat, Mariusz

(57) **Abstract**

The object of the invention is a cosmetic composition in the form of an oil-in-water emulsion comprising an antioxidant and a strong moisturising agent characterised in that it consists of an aqueous phase in the form of structural water from apples and an oil phase and an emulsifier, wherein active ingredients of the composition are ingredients selected from the group of: betulin, bakuchiol, glycerine-water extract from tomato, and/or glycerine-water extract from ginkgo, honey, acerola extract or mixtures thereof. An object of the invention is also a method of manufacturing of thereof.

## Description

The present invention relates to a cosmetic composition comprising an antioxidant and a strong moisturising agent. The resulting composition based on organic water is characterised by neutralisation of free radicals, deep moisturisation of the skin and stimulation of skin recovery and regeneration.

A cosmetic preparation for skin care is known from document PL412027. Said preparation comprises: hydro-alcoholic extract of freeze-dried elderflowers and/or extract obtained by supercritical CO₂ extraction, linseed oil, berry seed oil obtained by supercritical CO₂ extraction, isoamyl laurate, glycerine, mixture of glycerine stearate, cetaryl alcohol and polyglycerol-6 esters with palmitic and succinic acids, a mixture of sucrose esters of coconut oil and sorbitan stearate, thickeners, preservatives, antioxidants and, depending on the intended use, fragrances and water, wherein consistency regulator being xanthan gum, the antioxidant being tocopheryl acetate and/or ascorbyl palmitate, the preservative being sodium benzoate.

The aim of the invention was to develop a formulation of a cosmetic composition characterised by the neutralisation of free radicals, deep hydration of the skin and stimulation of skin recovery and regeneration.

Skin that undergoes natural ageing processes, i.e. through a decrease in telomerase activity, presents above all a shallowing of successive layers and a selective decrease in type I collagen (there are four types of collagen). Skin attacked by external factors, on the other hand, presents primarily a decrease in the volume of the extracellular matrix and fibre quality defects - e.g. fragmentation of collagen fibres, or the accumulation of abnormally formed elastin fibres. Macroscopically, this manifests itself primarily as an uneven loss of elasticity and loss of volume - furrows and wrinkles are formed, and, due to the melanocyte disorders that occur under these conditions, pigmentation and pigmented moles.

When the body is exposed to ultraviolet radiation, damage is caused primarily to DNA, which is fragmented under its influence. Free radicals are also spontaneously formed, causing peroxidation of membrane lipids as well as proteins. The fragments of cell components produced by these processes are referred to as DAMPs (damage-associated molecular patterns) - they have the ability to stimulate keranocytes to initiate a cascade of immune system activation via the chemokines TNF-α, IL-1β and IL-6. Through migrating Langerhans cells, they stimulate T helper lymphocytes and NK cells, which accumulate in damaged tissues, indicating ongoing repair processes. However, prolonged inflammation does not benefit the tissue as a whole, so anti-ageing cosmetics are designed to modulate the inflammation that occurs. The most important tasks also include activating cell repair genes, neutralising free radicals, but also inactivating UV-stimulated metalloproteinases - enzymes responsible for breaking down tissue components such as elastin fibres.

Therefore, the proposed line of phytocosmetics, based on organic apple water and key active ingredients, i.e. betulin, honey, bakuchiol, carnosine, natural oils and plant extracts, will uniquely neutralise free radicals, deeply hydrate the skin and stimulate its recovery and regeneration.

The object of the invention is a cosmetic composition in the form of an oil-in-water emulsion comprising an antioxidant and a strong moisturizing agent characterized in that it consists of an aqueous phase in the form of structural water from apples and an oil phase and an emulsifier, wherein active ingredients of the composition are ingredients selected from the group of: betulin, bakuchiol, glycerin-water extract from tomato, and/or glycerin-water extract from ginkgo, honey, acerola extract or mixtures thereof.

Advantageously, the oil phase is a mixture of glycerine and xanthan gum.

Beneficially, it additionally comprises as active substances: carrot seed oil, raspberry seed oil, Shea butter, aloe vera juice, orange extract, vitamin E acetate, hyaluronic acid 1%, argan oil, squalane, carnosine.

Advantageously, it additionally comprises an emollient, advantageously isoamyl laurate and/or isostearyl isostearate.

Advantageously, it additionally comprises preservatives, advantageously glycerol caprylate and/or glyceryl undecylenate and/or rosemary oil,
Advantageously, the emulsifier is selected from the group consisting of Glyceryl stearate, Polyglyceryl-6 palmitate, Polyglyceryl-6 succinate, Cetearyl alcohol, Cetearyl olivate, Sorbitan olivate or mixtures thereof.

Beneficially, it comes in the form of a lotion, mousse, serum or cream.

A further object of the invention is a method of manufacturing of a cosmetic composition described in claim 1, characterised in that organic water from apples is heated to 40°C, while in a separate vessel an appropriate amount of glycerine and xanthan gum is weighed out, mixed thoroughly, added to a mixer with organic water, resulting mixture is then meshed until gelling and heated to 75°C; during this time, weighed raw materials of oil phase are mixed in a melting tank, heated to 75°C and combined with the aqueous phase, and then homogenised for at least 10 minutes at a homogeniser speed of 5 000 to 10 000 rpm; resulting mass is then cooled to 40°C and active ingredients are then added while stirring and homogenised for 5 to 7 minutes at a speed of 5 000 to 10 000 rpm and cooling is continued until room temperature is reached; pH is measured afterwards.

The key ingredients in the composition are: organic water from apples, betulin, honey, bakuchiol, tomato extract (due to melatonin), carnosine, carrot seed oil, raspberry seed oil, orange extract, squalane, aloe vera, acerola extract (due to vitamin C), hyaluronic acid, argan oil, rosemary oil, ginkgo leaf extract.

The above-mentioned raw materials constitute a unique composition of natural raw materials, including those derived from up-cycling (concerning: organic water from apples, betulin extracted from birch bark (waste from wood production), raspberry seed oil (waste from the production of fruit juices), which in combination provide accumulated antioxidant, moisturising and skin-restructuring properties.

### Organic/structured apple water (condensate) - as a base for cosmetics.

It is the most widespread and also the most important chemical compound on the planet. It is the only substance that exists in three states of aggregation. As a liquid and ice, water covers 70% of the Earth's surface and, as water vapour, it is an essential component of the surrounding atmosphere. It plays a huge role in nature. It is essential for the life processes of living organisms and plants, e.g. in photosynthesis, in geochemical processes such as rock weathering. Water, thanks to its high heat capacity (storing a huge amount of heat), protects living organisms from overheating and cold, and also, due to its unusual temperature expansion, the surface layer of water does not sink when freezing, but remains on the surface - thanks to this, bodies of water do not freeze all the way to the bottom, which is of great importance for organisms overwintering in water.

The role of water in human life is equally enormous. There is about 75% of it in the human body, so it is obvious that the body's water supply must be constantly replenished. Although it is not a nutrient, without it life would not exist.

Water is directly consumed by us as an ingredient in drinks and food. The water content of the human body is between 50-75% of body weight. A reduction in body water content by 20% can lead to death. Water is also of fundamental importance in medicine and cosmetics. It is one of the elementary substances that form the basis of cosmetics and medicines. Together with the active ingredients dissolved in it, it is used in many medical, hygienic and cosmetic procedures.

Organic water from fruits is produced during the fruit juice concentration process using multi-stage evaporation stations. The fruit juice resulting from the mechanical pressing of the fruit is pasteurised and then concentrated in multi-stage evaporation stations. The evaporation of water from the juice (under conditions of reduced pressure and therefore reduced temperature) produces:
∘ concentrated fruit juice as the main product. Concentrated juice purchased by beverage bottlers for the production of reconstituted juices and fruit drinks.
∘ however, from the separation in the juice concentration process, the fruit water constitutes waste, which is sent to the on-site wastewater treatment plant. This treatment of fruit water is a great loss - because of the all-natural origin of this water and because of its properties. It is also important to note the global water shortage and the fact that, according to United Nations estimates, the availability of clean and safe drinking water could decrease by as much as 40% over the next decade - during the peak season, our plant produces approximately 400 tonnes of fruit water a day!

Structured water from apples, which is a waste product in the process of production of apple fruit concentrates will be used in cosmetics- upcycling. Characteristics of the quality sensory quality of apple condensates were described in the work by Bary 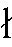 ko-Pikielna N., Matuszewska I., Szczecińska A., Radzankowska J., Characteristics of sensory quality of apple flavour condensates obtained from raw material of varied technological quality and varietal composition, Żywność, 2(23), 2000.

Water in the body is considered to be structured water - pure, natural, unprocessed water with beneficial properties. Proponents of the structural water theory believe that water is divided into "dead" and "living" water. So-called "dead" water flows in pipes and undergoes many purification processes that destroy its structure and contain calcium, iron and chlorine, which are harmful to the body. According to them, mineral water is also unhealthy, as it contains salts and minerals that are difficult for the body to assimilate. Due to the presence of these substances, the body has to purify and process it, resulting in the consumption of a large amount of energy that could be devoted to other purposes, such as cell regeneration.

Living water, on the other hand, is the vapour that falls to the ground in the form of rain and snow, then freezes, melts and flows into rivers and streams - such water is completely natural and has the closest structure to water present in the body, and is therefore better absorbed by it. The beneficial properties of structured water lie in its structure - not only is it better assimilated than ordinary bottled or tap water, but it also has a greater ability to dissolve and remove toxins and harmful substances from the body, promote the delivery of oxygen and nutrients to the cells. Because the body does not need much energy to assimilate it, it rapidly rehydrates, as well as regulating metabolic processes, stimulating liver function and facilitating intestinal cleansing.

Water activity takes values from 1 - for pure water, to 0 for an environment where there is no water or the water molecules do not have the ability to do the work (e.g. structured water). In general, the water activity of moist foods is between 1.00 - 0.90, medium water content foods has water activity between 0.90 and 0.55, and low water content foods has water activity between 0.55 and 0.00.

The introduction of the concept of water activity has made it possible to relate the thermodynamic state of water in food to its properties, quality and sustainability.

The role of water in cosmetics is extremely important, as it acts as a kind of solvent for active substances and is their carrier. In addition, water itself should moisturise the epidermis. Water is a carrier of information, it has a molecular memory, which was proven by the French immunologist Jacques Benveniste in 1988 and confirmed later in many experiments by, among others, the Russian Bakhir [Tony Edwards: Jacques Benveniste. [in:] The Independent [online]. 2004-10-10 [accessed 2018-03-18]. Philip Ball: The memory of water. [in:] 'Nature' [online]. Macmillan Publishers Limited, 2004-10-08 [accessed 2018-03-18]. Pawel Walewski: Brits lose homeopathy reimbursement from the state. At last! [in:] 'Polityka'. [online]. POLITYKA Sp. z o.o. S.K.A, 2018-03-14 [accessed 2018-03-18]. The founder of homeopathy Samuel Hahnemann was the precursor of water memory research. The issue was also addressed by Austrian natural scientists: Viktor Schauberger, Johannn Grander and, among others, the Japanese scientist Masaru Emoto, the Russian S. Zenin and others. The nervous system transmits only part of the information, while other information, e.g. about an organ's need for nutrients, is carried by water. Structured water is included in fresh fruit and vegetables. It can be obtained from melted ice or snow. It is this kind of water - from freshly squeezed juices or freshly melted snow - that has a rejuvenating and healing effect. The water has information about the plant from which the juice was squeezed. The plant, in turn, has information that flowed to it from the outside world: the time of day and night, the season, the abundance, structure and content of the soil, the strength of the magnetic field, the position of the sun and stars, even the people who grew the plant, and so on. Structured water has a unique arrangement of water molecules into water clusters that are smaller and arranged in a specific form, which gives structured water unique properties such as increased solubility, better hydration potential and lower genotoxicity.

Due to the specific form of the water molecule clusters, structured water penetrates more easily into the body's cells thus improving hydration.

Structured water has a lower level of genotoxicity which means that the potential toxins in the water have less harmful effects on the body's cells.

Structured water from the fruit is a pure, natural raw material; moreover, its use in cosmetics will reduce waste from the production of apple concentrates.

### Producing organic water from apples - the process flow:

Fruit water is produced during the process of concentrating fruit juices using multi-stage evaporation stations.
- The fruit juice resulting from the mechanical pressing of the fruit is pasteurised and then concentrated in multi-stage evaporative stations.
- The evaporation of water from the juice (under reduced pressure and therefore reduced temperature) produces:
   - concentrated fruit juice as the main product. Concentrated juice purchased by beverage bottlers for the production of reconstituted juices and fruit drinks.
   - whereas the fruit water separated during the juice concentration process is waste and sent to the on-site wastewater treatment plant. It is also possible to direct the water to a rectification station, where the natural fruit flavour is recovered and only the deacidified water is directed to the on-site treatment plant.

### Key active ingredients of the composition according to the invention:

• Betulin and honey - Have antioxidant as well as soothing and anti-inflammatory properties. The birch bark extract shows a very high value of total antioxidant activity, meaning that it is effective in neutralising free radicals and reactive oxygen species. The ATT for the pure honey solution is almost 20%, while the combination of both components (in the concentrations used in the cosmetic formulations later in this study) allows a higher efficiency (92.05%) of free radicals. This is a very high value of antioxidant activity, providing effective protection against oxidation of skin structures, which results in delaying the skin ageing process.
• Bakuchiol - a natural alternative to retinol, with anti-ageing effects. Meroterpene extracted from Psolarea corylifolia is a chemical compound with a partial terpenoid structure. The fruit of Psolarea corylifolia, known as Bakuci in various Ayurvedic pharmacopoeias, is a raw material known in traditional Chinese medicine, used for osteoporosis, among other things. It contains many compounds belonging to Bakuchiol: a successor to the retinol groups of flavonoids, coumarins or terpenes, many of which are potent antioxidants. Bakuchiol is not the most potent antioxidant, but it stands out because it is a functional analogue of retinol with a different structure.
Bakuchiol's strongest advantage over retinol is its photostability - although it exhibits similar effects, it is devoid of retinol's irritant properties and can even be used on extremely sensitive skin. In addition, bakuchiol, although it induces RAR (receptors for retinoic acid) receptors less strongly than retinol (it does not deliver typical retinoid metabolites due to its different structure), it induces genes encoding CRBP family proteins more strongly - for this reason, it increases the bioavailability of retinol, which can give tangible benefits in combination formulations. Bakuchiol is a retinol analogue in terms of RAR receptor activation, but manifests slightly different properties. The main difference is the antioxidant power of bakuchiol, for which there is only limited evidence in the case of retinol. Bakuchiol's ability to neutralise free radicals is due to the presence of a double bond at the phenol ring in the molecule - so that the resulting radical is stabilised by resonance.
An important component of skin inflammation that accelerates skin ageing is the local production of nitric oxide by nitric oxide synthase. By inhibiting NF-κB (nuclear factor kappa light chain enhancer of activated B cells), both compounds are able to reduce its production, slowing skin ageing. Greater differences in the potency of the compounds are observed in the induction and inhibition of the synthesis of proteins important in skin repair processes, including enzymes, the expression of which was studied on the skin substitute EpiDermFT. Bakuchiol shows a much greater ability to inhibit enzymes from the metalloproteinases group - it inhibits elastase almost nine times more strongly than retinol, while it inhibits collagenase, which is not affected at all by retinol, with 50% effectiveness. This translates into better results in tissue matrix reconstruction. Both compounds stimulate, at similar levels, the expression of aquaporin 3(AQP3), a channel protein that is responsible for regulating water/glycerol transport, which helps to maintain normal skin hydration levels, provide elasticity and barrier repair. In addition, bakuchiol doubly potently induces one of the key genes associated with hydration and skin barrier homeostasis, E-cadherin (CDH1), a Herbalism protein responsible for the correct construction of intercellular tight junctions and the formation of a water barrier that allows water retention in the cell.

| gene | Description of the gene | Function | Bakuchiol | Retinol |
|---|---|---|---|---|
| AQP3 | Aquaporin 3 | Aquaporin 3 is a protein transport channel for water/glycerol that is expressed in the epidermis, helping to maintain proper skin hydration level, elasticity and restoration of the skin barrier | 4.3 | 3.5 |
| CDH1 | E-cadherin | E-cadherin is a key protein in the formation of the water barrier, is essential in properly formed tight joints | 21.6 | 9.4 |

In the search for a preparation to prevent and offset the effects of ageing, many studies are attempting to combine various antioxidants (e.g. vitamin C or melatonin) with phenolic substances, such as bakuchiol. The synergistic effect of the above-mentioned compounds has been proven. When vitamin C, melatonin and bakuchiol are used simultaneously, it has been found that there is an enhanced antioxidant effect, weaker observed when melatonin and bakuchiol are combined.
Pulsed optical radiolysis studies have confirmed the inhibitory effects of bakuchiol on: linoleic acid peroxyl radicals, DPPH (2,2-diphenyl-1-picrylhydrazyl) radicals and glucyl radicals. It has also been shown to prevent lipid and protein peroxidation. In addition, a synthetic derivative of bakuchiol (o-methyl bakuchiol) was found to have similar activities. O-methylbakuchiol inhibited lipid peroxidation in rat brain homogenates. Based on the study, it was concluded that the terpenoid chains present in the structure of both compounds (bakuchiol and its o-methyl derivative) promote their oxidative activity.
Chronic sun exposure disrupts the structural integrity of the upper epidermis and stratum corneum, alters its hydration and lipid properties, as well as its thickness. In the study, skin explants taken from the abdomen of women aged 30 and 32 years were subjected to one-hour UVA and UVB irradiation for four days and the reaction of the irritated skin to a formulation in which bakuchiol was combined with the antioxidants comprising melatonin and a vitamin C derivative. When this serum was used, a large increase in the expression of filaggrin, which provides protection against harmful agents and moisture loss, was noted. There was also an increase in the expression of laminin, the main component of basement membranes involved in the formation of its proper structure and stable connections.
In the analyses discussed above, it has been shown that the use of a combination of these compounds with regular application increases the expression of proteins that improve skin barrier function and hydration levels.
In another study, skin sections taken from the abdomen from two women were subjected to one-hour ultraviolet irradiation for four consecutive days with a serum of similar composition (vitamin C, melatonin, bakuchiol). One hour after irradiation, a serum consisting of vitamin C, melatonin and bakuchiol was applied. It was observed that the application of the serum restored the homeostatic properties of the photo-aged skin. An increase in HIF-1 α mRNA (hypoxia-induced factor) was observed, which regulates collagen biosynthesis, accelerates skin wound healing, stimulates angiogenesis and improves the skin barrier, and consequently increases skin firmness, inhibiting skin ageing.
• Carnosine - an antioxidant with protective properties for the skin. Carnosine is a dipeptide (a combination of two amino acids: beta-alanine and histidine) that occurs naturally in the human body, including the skin. It was first isolated and described in 1900 by Russian scientist Vladimir Gulevich (Gulewitsch).
With regard to the effect of carnosine on the skin, its wound-healing-accelerating properties are particularly noteworthy. Experimental studies have shown that carnosine reduces the levels of pro-inflammatory and profibrotic cytokines, which play an important role in the healing process. In other words, high levels of inflammatory cytokines are one of the factors contributing to wounds not wanting to heal.
Carnosine, being a source of amino acids such as histidine and alanine, regulates the formation of histamine (a substance involved in reparative, inflammatory and allergic processes) released during trauma by accelerating wound healing, which has been repeatedly confirmed in studies. In addition, it accelerates collagen production; it stimulates fibroblast cell division, connective tissue production and intracellular regeneration.
• Carrot seed oil - rich in beta-carotene, which acts as a natural UV filter. It contains valuable ingredients, including oleic acid, which speeds up regeneration processes. In turn, beta-carotene (provitamin A) protects the skin from the harmful effects of prolonged exposure to the sun's rays. In addition, it normalises sebum production. Thus, oily and problematic skin responds perfectly to the action of provitamin A.
• Orange extract - a substance with very broad properties used in cosmetics. Recommended for skin with inflammation, oily skin and enlarged pores, acne. It slows down ageing processes and premature wrinkles. It contains high concentrations of peptides, flavonoids, vitamins and minerals as well as AHA acids and essential oils. Orange stimulates cell regeneration and collagen synthesis, strengthens and protects fragile capillaries, has an anti-inflammatory and antibacterial effect. It moisturises and brightens the complexion. Properties:
∘ The antioxidant effect is due to the flavonoids, anthocyanins and vitamin C content;
∘ Stimulating cell regeneration;
∘ Stimulating collagen synthesis;
∘ Anti-inflammatory;
∘ Strengthening and protecting blood vessels;
∘ Reducing cell mitosis;
∘ Impact on the hair shaft;
∘ Antimicrobial action;
∘ Tyrosinase inhibition;
∘ Regulation of transepidermal water loss.
Active substances:
∘ Flavonoids - include hesperidin, naringin, luteolins and ferulic acid;
∘ AHA organic acids - mainly malic acid and citric acid;
∘ Anthocyanins - include glucoside-3-cyanidin and glucoside-3-delphinidin;
∘ Vitamins - the main vitamin contained in oranges is vitamin C. It also contains vitamin A, E and B vitamins;
∘ Essential oil;
∘ Carbohydrates - glucose, fructose and sucrose;
∘ Colours - carotenes and xanthophylls;
∘ Coumarins, diterpenes (6,7-dimethoxycoumarin), the bitter component of limonins, phytosterols and their esters, phenolic acids (hydroxycinnamic, ferulic, coumaric and caffeic acids), proteins, carotenoids, pectins.
• Ginkgo biloba extract - with a guaranteed flavonoid content of 24% and 6% terpene lactones. The extract has a stimulating and strengthening effect on the circulatory system, improves blood circulation, strengthens the vascular system and is a very strong antioxidant. It is mainly used for its strengthening of the connective tissue of the skin - it inhibits the skin ageing process. In addition to anti-aging products, it is commonly used in anti-cellulite, firming, eye shadow brightening and vascular skin care preparations.
• Squalane - a natural emollient, moisturising and firming.
• Raspberry seed oil - a natural UV filter, rich in fatty acids.
• Aloe vera - moisturises and soothes.
• Hyaluronic acid - deeply moisturises and improves skin elasticity.
• Argan oil - nourishes and protects the skin.
• Rosemary oil - has antibacterial and antioxidant properties.
• Tomato extract - has properties:
∘ Antioxidant - melatonin helps protect the skin from oxidative stress and damage caused by environmental factors;
∘ Anti-inflammatory - can help reduce inflammation and skin irritation;
∘ Supporting skin regeneration - melatonin can support the skin's repair processes, contributing to its overall health.
• Acerola extract - is one of the richest sources of vitamin C, which acts as a powerful antioxidant. Here are some of its main properties:
∘ Antioxidant - vitamin C neutralises free radicals, protecting the skin from oxidative stress;
∘ Brightening - can help reduce hyperpigmentation and even out skin tone;
∘ Supporting collagen production - vitamin C is key to collagen synthesis, helping to improve skin elasticity and reduce signs of ageing.

The essence of the invention is an organic water-based cosmetic composition comprising an antioxidant and a strong moisturising agent, characterised in that it comprises betulin, honey, bacuchiol, tomato extract, carnosine, carrot seed oil, raspberry seed oil, orange extract, squalane, aloe vera, acerola extract, hyaluronic acid, argan oil, rosemary oil, ginkgo leaf extract.

In one embodiment, the active ingredients of the body lotion are Natragem EW-FL-(MW) (2.00%), Olivem 1000 (3.00%), isoamyl laurate (3.00%), carrot seed oil (3.00%), raspberry seed oil (3.00%), Masso Care ISI (3.00%), Shea butter (5.00%), betulin (0.05%), vit. acetate. E (0.50%), aloe vera juice (3.00%), bakuchiol (1.00%), tomato glycerine-water extract (2.00%), ginkgo-water extract (2.00%), acerola extract (2.00%), honey (3.00%), orange extract (4.00%), Lexgarf Natural MB (1.50%), rosemary oil (0.15%).

In one embodiment, the active ingredients in the face mousse are Olivem 1000 (4.50%), Natragem EW-FL-(MW) (2.80%), carrot seed oil (3.00%), squalane (3.00%), argan oil (4.00%), isoamyl laurate (5.00%), tocopherol acetate (0.50%), betulin (0.05%), honey (2.00%), aloe vera (2.00%), bakuchiol (1.00%), tomato glycerine-water extract (2.00%), ginkgo-water extract (2.00%), acerola extract (2.00%), hyaluronic acid 1% (3.00%), orange extract (2.00%), Lexgarf Natural MB (1.50%), rosemary oil (0.15%).

In one embodiment, the active ingredients of the facial serum are Natragem EW-FL-(MW) (2.80%), Olivem 1000 (4.50%), argan oil (3.50%), squalane (3.00%), Masso Care ISIS (3.00%), raspberry seed oil (2.00%), cetyl-styryl alcohol (1.75%), betulin (0.05%), vit. E (0.50%), water (3.00%), carnosine (0.10%), bakuchiol (1.00%), tomato glycerine-water extract (2.00%), ginkgo-water extract (2.00%), acerola extract (2.00%), Lexgarf Natural MB (1.50%), sodium hyaluronate 1% (2.50%), rosemary oil (0.15%).

In one formulation, the active ingredients of the eye mousse are Olivem 1000 (3.00%), squalane (5.00%), raspberry oil (1.00%), argan oil (3.00%), vit. acetate. E (0.30%), betulin (0.01%), hyaluronic acid S (0.30%), carnosine (0.10%), water (4.00%), aloe vera (3.00%), bakuchiol (1.00%), tomato glycerine-water extract (2,00%), glycerol-water extract of ginkgo (2.00%), acerola extract (2.00%), Lexgarf Natural MB (1.50%), rosemary oil (0.15%).

All products are in the form of a stable oil-in-water emulsion with microbiological purity in accordance with legal requirements, dermatological tests did not reveal any positive allergic reactions during application and instrumental tests conducted on a group of probationers confirmed the cosmetic's moisturising and smoothing effects.

The proposed cosmetic products have a well-balanced formulation that provides effective moisturisation, antioxidant protection and anti-ageing action. Thanks to various emollients and humectants, the products moisturise the skin and prevent it from drying out. Anti-inflammatory and antioxidant ingredients help protect the skin from oxidative stress, while active ingredients such as bakuchiol promote collagen production to help fight the signs of ageing. **In** addition, preservatives ensure the shelf life of the product, while rosemary oil adds a delicate fragrance and has an antibacterial effect.

### Benefits of the proposed composition:

### 1. Using organic (structured) water from apples as a base.

Use of organic water from apples as a product base: there are currently no cosmetic products on the market that are based on organic water from up cycling (the water is extracted during the production of fruit concentrates and is a production waste for the production process).

Water in the body is considered to be structured water - pure, natural, unprocessed water with beneficial properties. Proponents of the structural water theory believe that water is divided into ",dead" and "living" water. So-called "dead" water flows in pipes and undergoes many purification processes that destroy its structure and contain calcium, iron and chlorine, which are harmful to the body. According to them, mineral water is also unhealthy, as it contains salts and minerals that are difficult for the body to assimilate. Due to the presence of these substances, the body has to purify and process it, resulting in the consumption of a large amount of energy that could be devoted to other purposes, such as cell regeneration.

Living water, on the other hand, is the vapour that falls to the ground in the form of rain and snow, and is extracted from fruit and vegetables - such water is completely natural and has the closest structure to water present in the body, and is therefore better absorbed by it. The beneficial properties of structured water lie in its structure - not only is it better absorbed than ordinary bottled or tap water, but it also has a greater ability to dissolve and remove toxins and harmful substances from the body, promoting the delivery of oxygen and nutrients to the cells.

Structured water, allows the body's electrolyte balance to be restored. The change in the state of aggregation of water (e.g. the benefits of rainwater), results in the 'ordering' of its molecules, thus making them similar to those found in the cells of plants and living organisms. This is why vegetables and fruit provide biologically active water to our body, which is so beneficial. Such water hydrates much better and restores the water-salt balance of our body.

The structure of living water is completely different from the water we are used to (bottled, unfiltered from the tap). Alkaline water is made up of bipolar molecules linked by hydrogen bonds and forming so-called associations, i.e. groups made up of 5 - 6 atoms (which accounts for the faster penetration of alkaline water and the beneficial minerals it contains into our body cells, as well as the faster elimination of free radicals and faster detoxification).

### 2. Combine Bakuchiol with acerola extract (for its vitamin C content) and tomato extract (for its melatonin content) to achieve a synergistic antioxidant effect.

Bakuchiol in combination with acerola extract (for its vitamin C content) and tomato extract (for its melatonin content) : As studies show, the combination of vitamin C, melatonin and bakuchiol results in enhanced antioxidant effects. Pulsed optical radiolysis studies have confirmed the inhibitory effect of bakuchiol on: linoleic acid peroxyl radicals, DPPH (2,2-diphenyl-1-picrylhydrazyl) radicals and glucyl radicals. It has also been shown to prevent lipid and protein peroxidation. In addition, a synthetic derivative of bakuchiol (o-methyl bakuchiol) has been found to have similar effects. O-methylbakuchiol inhibited lipid peroxidation in rat brain homogenates. Based on the study, it was concluded that the terpenoid chains present in the structure of both compounds (bakuchiol and its o-methyl derivative) promote their oxidative activity.

Chronic sun exposure disrupts the structural integrity of the upper epidermis and stratum corneum, alters its hydration and lipid properties, as well as its thickness. In the study, skin explants taken from the abdomen of women aged 30 and 32 years were subjected to one-hour UVA and UVB irradiation for four days and the reaction of the irritated skin to a formulation in which bakuchiol was combined with the antioxidants comprising melatonin and a vitamin C derivative. When this serum was used, a large increase in the expression of filaggrin, which provides protection against harmful agents and moisture loss, was noted. There was also an increase in the expression of laminin, the main component of basement membranes involved in the formation of its proper structure and stable connections.

In the analyses discussed above, it has been shown that the use of a combination of these compounds with regular application increases the expression of proteins that improve skin barrier function and hydration levels.

In another study, skin sections taken from the abdomen from two women were subjected to one-hour ultraviolet irradiation for four consecutive days with a serum of similar composition (vitamin C, melatonin, bakuchiol). One hour after irradiation, a serum consisting of vitamin C, melatonin and bakuchiol was applied. It was observed that the application of the serum restored the homeostatic properties of the photo-aged skin. An increase in HIF-1 α mRNA (hypoxia-induced factor) was observed, which regulates collagen biosynthesis, accelerates skin wound healing, stimulates angiogenesis and improves the skin barrier, and consequently increases skin firmness, inhibiting skin ageing.

### 3. Inclusion of betulin and honey in the formulation to ensure maximum antioxidant and skin regenerating effect

The object of the invention and the method of manufacturing thereof are shown in the following examples.

### Example I. Characteristics of birch bark extract

For the dry birch bark extract obtained after solvent evaporation, physicochemical properties were determined and quantitative and qualitative analysis (for betulin content) was performed (using liquid chromatography, HPLC). An antioxidant activity test was performed using the DPPH stable radical reduction method. The irritant potential of the extract *was evaluated* in vitro, by the colorimetric method (MTT), using the reconstructed epidermis model "EpiDerm^{®} (MatTek Corporation). The results were used to develop a REACH-compliant material safety data sheet.

Analysis carried out using an Agilent 1100 series HPLC liquid chromatograph, with UV detector, on a ThermoScientific ODS Hypersil 250x4.6 (5um) column, using a gradient programme: 0-5min 80% acetonitrile (ACN), 20% water (H2O) with 0.1% trifluoroacetic acid (TFA), 5-12 min 100% ACN, 12-27 min 100% ACN, injection of 100 microlitres A flow rate of 1ml/min was used.

The extract was analysed for its triterpene fraction (betulin, betulinic acid and lupeol content). Figure 1 shows the chromatogram of the birch bark extract. The presence of three main triterpenes - betulin, betulinic acid and lupeol - was identified in the birch bark extract samples. Table 1 shows the percentage content of each triterpene determined from the areas of the peaks on the chromatogram. The measurement refers to a wavelength of 210 nm.

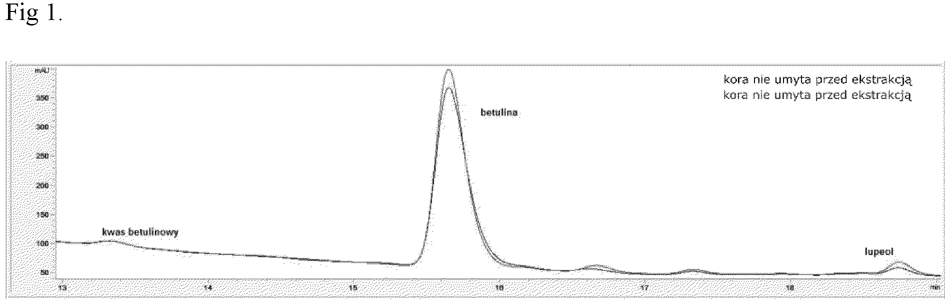

**Table 1.**

| Name of substance | Content [%] |
|---|---|
| Betulin | 86,16 |
| Betulinic acid | 2,07 |
| lupeol | 0,92 |
| Other relationships | 10,85 |

### Example II.

The total antioxidant activity (TAA) of betulin (birch bark extract) was determined using a modified Brand-Williams *method with* the DPPH (2,2-diphenyl-1-picrylhydrazyl) radical. The antioxidant activity of the sample is analysed by neutralising the coloured radical to colourless compounds. The colour change is proportional to the antioxidant content and activity of the sample. Quantitatively, the degree of decolourisation of the reagent solution is measured spectrophotometrically at 515 nm. Absorbance measurements were made using a Nanocolor UV/VIS spectrophotometer from Macherey Nagel. The measurement temperature was 20°C. Three determinations each were made for the samples tested (the result is the average value of the three measurements). The value of the total antioxidant activity of the product was expressed as a percentage of DPPH radical inhibition compared to the reference sample.

Table 2 shows the results of the evaluation of the total antioxidant activity for birch extract at concentrations of 0.25 per cent and 0.5 per cent, as well as for honey at 5 per cent by weight, which corresponds to the concentrations used in the cosmetic preparations developed within the project.

**Table 2.**

| Active substance (concentration in solution) | TAA value [% inhibition of oxidation processes]. | Standard deviation |
|---|---|---|
| Birch extract (0.25%) | 85.82 | 0.31 |
| Birch extract (0.5%) | 89.94 | 0.81 |
| Honey (5%) | 19.55 | 2.26 |
| Birch extract 0.5% + honey 5% | 92.05 | 0.15 |

### Example III.

In order to determine the safety of birch extract in cosmetics, a method was used to determine the irritant potential using the Epiderm human skin model, which contains a multilayered differentiated epidermis. In this method, the exposure time at which a 50% reduction in the number of viable cells (ET50) occurs is assessed. This number is assessed by measuring the reduction of MTT - 3-(4,5-dimethylthiaz-2-yl)-2-5-diphenyltetrazolium bromide). Formazan, which is blue-violet in colour, is formed as a result of mitochondrial metabolism. Formazan formation allows assessment of the number of cells that have been damaged. A chemical is classified as an irritant if the cell survival rate relative to a negative sample (containing neutral solvent) is below 50%.

The test consisted of applying an appropriate amount of a solution of the test compound to the surface of the epidermis model. Birch extract solutions of 0.25 per cent and 0.5 per cent by weight were prepared, corresponding to the content of this active ingredient in cosmetic preparations. The procedure for measuring the potential irritant potential was performed according to the SOP (Standard Operation Protocol) of ECVAM [https://jeodpp.jrc.ec.europa.eu/ftp/jrc-opendata/EURL-ECVAM/datasets/ThyroidMethods/2a/Method%202a_SOP_Assay_Part%202_JRC133079.pd f]. A positive sample (5% SLS) and a negative sample, which was phosphate buffer (pH 7.4), were also tested simultaneously with the extract solutions. Three measurements were taken for each sample tested. The cell viability scores were compared against the negative control. Table 3 shows the results obtained.

**Table 3.**

| Test ingredient | Relative cell viability [%] | Result: irritant/non-irritant |
|---|---|---|
| Solution of 0.25% birch bark extract | 86.20 | non-irritating |
| Solution of 0.5% birch bark extract | 90.02 | non-irritating |
| Positive control (5% SLS*) | 6.03 | irritating |
| Negative control (phosphate buffer pH=7.4) | 100.00 | non-irritating |

### Example IV.

The qualitative and quantitative composition of the base body lotion formulation is shown in Table 4.

**Table 4.**

| No. | Phase | INCI | content % |
|---|---|---|---|
| 1 | w | Organic water from apples | 62.60 |
| 2 | w | Xanthan gum | 0.20 |
| 3 | w | Glycerine | 3.00 |
| 4 | o | glyceryl stearate*, polyglyceryl palmitate/succinate-6*, cetearyl alcohol * | 2.00 |
| 5 | o | Cetearyl olivate, Sorbitan olivate | 3.00 |
| 6 | o | Isoamyl laurate | 3.00 |
| 7 | o | Daucus Carota Sativa Seed Oil (Daucus Carota Sativa Seed Oil) | 3.00 |
| 8 | o | Rubus Idaeus Seed Oil (Rubus Idaeus Seed Oil) | 3.00 |
| 9 | o | Isostearyl Isostearate (Isostearyl isostearate) | 3.00 |
| 10 | o | Butyrospermum Parkii Butter (Butyrospermum Parkii Butter) | 5.00 |
| 11 | o | Betulin (betulin) | 0.05 |
| 12 | o | Tocopherol Acetate (Tocopheryl acetate) | 0.50 |
| 13 | 40 | Aloe Barbadensis Leaf Juice (Aloe Barbadensis leaf juice) | 3.00 |
| 14 | 40 | Bakuchiol | 1.00 |
| 15 | 40 | glycerine-water extract from tomato | 2.00 |
| 16 | 40 | Glycerine-water extract from ginkgo biloba | 2.00 |
| 17 | 40 | Acerola extract | 2.00 |
| 17 | 40 | honey | 3.00 |
| 18 | 40 | Orange extract | 4.00 |
| 19 | 40 | Glycerol caprylate (i) glyceryl undecylenate | 1.50 |
| 20 | 40 | Rosemary oil | 0.15 |

### The role of the composition component:

Water is the main ingredient in the lotion and forms the basis of its texture. In this case, the water comes from organic apples, which can provide additional nutrients and antioxidants.

Xanthan gum is a thickener and stabiliser that helps maintain the consistency of the lotion and prevents the oil and water phases from separating.

Glycerine is a powerful humectant, meaning that it attracts moisture to the skin, helping to moisturise it.

Natragem EW-FL-(MW) (glyceryl stearate*, polyglyceryl-6 palmitate/succinate*, cetearyl alcohol*) is an emulsifier that maintains the stability and homogeneity of the lotion.

Olivem 1000 (Cetearyl Olivate, Sorbitan Olivate) is an emulsifier that is of natural origin. It has a gentle effect on the skin and improves the texture of the lotion.

Isoamyl laurate is a light emollient that gives the lotion a silky texture.

Carrot seed oil is rich in beta-carotene, which can act as a natural UV filter and improve skin elasticity.

Raspberry seed oil contains fatty acids and has moisturising and protective properties, also as a natural UV filter.

Isostearyl Isostearate is an emollient, meaning that it helps to moisturise and soften the skin. Its ability to form a soft, protective layer on the skin's surface helps prevent moisture loss, leaving the skin smooth and soft.

Rich in fatty acids and vitamins, shea butter nourishes and moisturises the skin, helping it to regenerate.

Betulin, an ingredient derived from birch bark, has anti-inflammatory effects and can promote skin regeneration. Strong antioxidant effect.

Vitamin E acetate is a powerful antioxidant that helps protect the skin from free radical damage. Aloe vera juice has a moisturising and soothing effect, ideal for sensitive skin. It has anti-inflammatory and antioxidant effects.

Bakuchiol is a natural compound derived from plants, known for its anti-ageing properties. It is a mild substitute for retinol, helping to stimulate collagen production and reduce the appearance of wrinkles.

Tomatoes contain lycopene, which has an antioxidant effect, helping to protect the skin from oxidative damage. In addition, due to its melatonin content, it has an effect:
- Antioxidant: Melatonin helps protect the skin from oxidative stress and damage caused by environmental factors.
- Anti-inflammatory: May help reduce inflammation and skin irritation.
- Supporting skin regeneration: Melatonin can support the skin's repair processes, contributing to its overall health.

Ginkgo (Ginkgo biloba) has anti-inflammatory and antioxidant properties and improves blood circulation in the skin.

Acerola is one of the richest sources of vitamin C, which acts as a powerful antioxidant. Here are some of its main properties:
- Antioxidant: Vitamin C neutralises free radicals, protecting the skin from oxidative stress.
- Brightening: Can help reduce hyperpigmentation and even out skin tone.
- Supporting collagen production: Vitamin C is key to collagen synthesis, helping to improve skin elasticity and reduce signs of ageing.

Honey is a natural humectant that moisturises the skin and also has antibacterial properties. Orange extract adds freshness and aroma to the lotion, and also has antioxidant properties. Glycerol caprylate (and) glyceryl undecylenate are natural preservatives that protect the lotion from bacteria and fungi.

Rosemary oil adds a fresh fragrance to the lotion and acts as a natural preservative, also having antibacterial properties.

### Example V.

The technological process of the body lotion recipe consists of the following steps:
1. In the main mixer, heat the weighed amount of organic water from the apples to 40°C;
2. In a separate vessel, weigh out the appropriate amount of glycerine and xanthan gum. Mix the whole thoroughly, add to a mixer with organic water. Stir until gelling, then heat the whole mixture to 75°C;
3. Mix the weighed raw materials of the oil phase together in a melting tank, heat to 75°C;
4. Pour the oil phase ingredients into the water phase (into the mixer). Homogenise for about 15 minutes, homogeniser speed 5000-10000 rpm;
5. Cool the resulting mixture to about 40°C and then add the active ingredients, stirring all the time;
6. Once all the active ingredients have been added, homogenise the whole (about 5-7 minutes, speed 5000-10000 rpm). Continue cooling until room temperature is reached;
7. Measure pH, adjust pH if necessary;
8. Collect a sample for microbiological testing and for archiving.

### Example VI.

The qualitative and quantitative composition of the base formulation of the facial mousse is shown in Table 5.

**Table 5.**

| No. | Phase | INCI | content % |
|---|---|---|---|
| 1 | w | Aqua organic water from apples | 63.30 |
| 2 | w | Xanthan Gum | 0.20 |
| | | Xanthan gum | |
| 3 | w | Glycerin | 3.00 |
| | | Vegetable glycerine of pharmaceutical quality 99.5% | |
| 4 | o | Cetearyl Olivate*, Sorbitan Olivate*. | 4.50 |
| 5 | o | Glyceryl Stearate*, Polyglyceryl-6 Palmitate/ Succinate*, Cetearyl Alcohol*. | 2.80 |
| 6 | o | Daucus Carota Sativa Seed Oil | 3.00 |
| | | Carrot seed oil | |
| 7 | o | Squalane | 3.00 |
| | | Squalane | |
| 8 | o | Argania Spinosa Kernel Oil | 4.00 |
| | | Argan oil | |
| 9 | o | Isoamyl Laurate | 5.00 |
| | | Isoamyl laurate | |
| 10 | o | Tocopherol Acetate | 0.50 |
| | | Tocopheryl acetate | |
| 11 | o | Betulin | 0.05 |
| | | Betulin | |
| 12 | 40 | Mel | 2.00 |
| | | Honey | |
| 13 | 40 | Aloe Barbadensis Leaf Extract | 2.00 |
| | | Aloe vera | |
| 14 | 40 | Bakuchiol | 1.00 |
| | | Bakuchiol | |
| 15 | 40 | glycerine-water extract from tomato | 2.00 |
| 16 | 40 | Glycerine-water extract from ginkgo biloba | 2.00 |
| 17 | 40 | Acerola extract | 2.00 |
| 18 | 40 | Sodium Hyaluronate, Aqua | 3.00 |
| | | Hyaluronic acid 1% | |
| 19 | 40 | Orange extract | 2.00 |
| 20 | 40 | Glyceryl Caprylate (and) Glyceryl Undecylenate | 1.50 |
| 21 | 40 | Rosemary oil | 0.15 |

The role of compositional components as in example IV and additionally:
Squalane is a stable form of squalene, a natural lipid found in human skin. It acts as an excellent emollient, helping to moisturise the skin and prevent water loss through the epidermis. It is lightweight, absorbs well and does not clog pores.

Antioxidant properties: Squalane can help protect the skin from environmental damage and oxidative stress.

Argan oil contains high concentrations of unsaturated fatty acids, such as oleic and linoleic acid, which helps to nourish the skin.

Anti-ageing and regenerating: Thanks to its vitamin E and other antioxidant content, argan oil can promote skin regeneration, reduce the appearance of fine lines and improve skin elasticity.

Argan oil is an excellent emollient that helps moisturise the skin and makes it smooth.

Hyaluronic acid is a powerful humectant that binds water in the skin, providing deep hydration. Improves skin elasticity: Thanks to its ability to retain water, hyaluronic acid can increase skin elasticity and firmness.

### Example VII.

The technological process of the facial mousse recipe follows Example V.

### Example VIII.

The qualitative and quantitative composition of the base formulation of the facial serum is shown in Table 6.

**Table 6.**

| No. | Phase | INCI | content % |
|---|---|---|---|
| 1 | w | Aqua organic water from apples | 63.40 |
| 2 | w | Xanthan Gum | 0.25 |
| | | Xanthan gum | |
| 3 | w | Glycerin | 3.00 |
| | | Vegetable pharmaceutical glycerine 99.5% | |
| 4 | o | Glyceryl Stearate*, Polyglyceryl-6 Palmitate/ Succinate*, Cetearyl Alcohol* | 2.80 |
| 5 | o | Cetearyl Olivate*, Sorbitan Olivate*. | 4.50 |
| 6 | o | Argania Spinosa Kernel Oil | 3.50 |
| | | Argan oil | |
| 7 | o | Squalane | 3.00 |
| | | Squalane | |
| 8 | o | Isostearyl Isostearate | 3.00 |
| 9 | o | Rubus Idaeus Seed Oil | 2.00 |
| | | Raspberry seed oil | |
| 10 | o | Ccetearyl Alcohol | 1.75 |
| | | Cetyl stearyl alcohol | |
| 11 | o | Betulin | 0.05 |
| | | Betulin | |
| 12 | o | Tocopherol Acetate | 0.50 |
| | | Vit. E acetate | |
| 13 | 40 | Aqua | 3.00 |
| 14 | 40 | Carnosine | 0.10 |
| | | Carnosine | |
| 15 | 40 | Bakuchiol | 1.00 |
| 16 | 40 | tomato glycerine-water extract | 2.00 |
| 17 | 40 | Glycerine-water extract of ginkgo biloba | 2.00 |
| 18 | 40 | Acerola extract | 2.00 |
| 19 | 40 | Glyceryl Caprylate (and) Glyceryl Undecylenate | 1.50 |
| 20 | 40 | Sodium Hyaluronate, Aqua | 2.50 |
| | | sodium hyaluronate 1% | |
| 21 | 40 | Rosemary oil | 0.15 |

Role of compositional components as in example IV, VI and additionally:
Raspberry seed oil is rich in fatty acids and has moisturising properties, and can also act as a natural UV filter.

Carnosine has antioxidant properties and can help delay the signs of ageing by protecting the skin from free radical damage.

### Example IX.

The technological process of the facial serum formulation is as in example V.

### Example X.

The qualitative and quantitative composition of the base formulation of the eye mousse is shown in Table 7.

**Table 7.**

| No. | Phase | INCI | content % |
|---|---|---|---|
| 1 | w | Aqua | 71.54 |
| | | organic water from apples | |
| 2 | w | Xanthan Gum | 0.10 |
| | | xanthan gum | |
| 3 | w | Glycerin | 2.00 |
| | | Vegetable glycerine of pharmaceutical quality | |
| 4 | o | Cetearyl Olivate*, Sorbitan Olivate*. | 3.00 |
| 5 | o | Sqalane | 5.00 |
| | | Squalane | |
| 6 | o | Rubus Idaeus Seed Oil | 1.00 |
| | | Raspberry oil | |
| 7 | o | Argania Spinosa Kernel Oil | 3.00 |
| | | Argan oil | |
| 8 | o | Tocopherol Acetate | 0.30 |
| | | Vitamin E acetate | |
| 9 | o | Betulin | 0.01 |
| | | Betulin | |
| 10 | 40 | Sodium Hyaluronate, Aqua | 0.30 |
| | | Hyaluronic acid S | |
| 11 | 40 | Carnosine | 0.10 |
| | | Carnosine | |
| 12 | 40 | Aqua | 4.00 |
| | | water | |
| 13 | 40 | Aloe Barbadensis Leaf Extract | 3.00 |
| | | Aloe vera | |
| 14 | 40 | Bakuchiol | 1.00 |
| | | Bakuchiol | |
| 15 | 40 | glycerine-water extract from tomato | 2.00 |
| 16 | 40 | Glycerine-water extract from ginkgo biloba | 2.00 |
| 17 | 40 | Acerola extract | 2.00 |
| 18 | 40 | Glyceryl Caprylate (and) Glyceryl Undecylenate | 1.50 |
| 19 | 40 | Rosemary oil | 0.15 |

### Role of composition components as in examples IV, VI and VIII.

### Example XI.

The technological process of the eye mousse recipe is as in example V.

## Claims

1. A cosmetic composition in the form of an oil-in-water emulsion comprising an antioxidant and a strong moisturising agent, **characterised in that** it consists of an aqueous phase in the form of structured water from apples and an oil phase and an emulsifier, wherein active ingredients of the composition are selected from the group of: betulin, bakuchiol, glycerine-water extract from tomato, and/or glycerine-water extract from ginkgo, honey, acerola extract or mixtures thereof.

2. The composition according to claim 1, **characterised in that** the oil phase is a mixture of glycerine and xanthan gum.

3. The composition according to claim 1, **characterised in that** it additionally comprises as active substances: carrot seed oil, raspberry seed oil, shea butter, aloe vera juice, orange extract, vitamin E acetate, hyaluronic acid 1%, argan oil, squalane, carnosine.

4. The composition according to claim 1, **characterised in that** it additionally comprises an emollient, preferably isoamyl laurate and/or isostearate.

5. The composition according to claim 1, **characterised in that** it additionally comprises preservatives, advantageously glycerol caprylate and/or glycerol undecylenate and/or rosemary oil.

6. The composition according to claim 1, **characterised in that** the emulsifier is selected from the group consisting of Glyceryl stearate, Polyglyceryl-6 palmitate, Polyglyceryl-6 succinate, Cetearyl alcohol, Cetearyl olivate, Sorbitan olivate or mixtures thereof.

7. The composition according to claim 1, **characterised in that** it is in the form of a lotion, mousse, serum or cream.

8. A method of manufacturing of a cosmetic composition according to claim 1, **characterised in that** organic water from apples is heated to a temperature of 40°C, while in a separate vessel an appropriate amount of glycerine and xanthan gum is weighed out, mixed thoroughly, added to a mixer with organic water, resulting mixture is then meshed until gelling and heated to 75°C; during this time, weighed raw materials of oil phase are mixed in a melting tank, heated to 75°C and combined with the aqueous phase, and then homogenised for at least 10 minutes at a homogeniser speed of 5 000 to 10 000 rpm; e resulting mass is then cooled to 40°C and active ingredients are then added while stirring and homogenised for 5 to 7 minutes at a speed of 5 000 to 10 000 rpm and cooling is continued until room temperature is reached; pH is measured afterwards.
